# EUROPÄISCHE PATENTSCHRIFT

(11) **EP 2 842 598 B1**
(45) Veröffentlichungstag und Bekanntmachung des Hinweises auf die Patenterteilung: **03.05.2017**
(21) Anmeldenummer: 14174639.6
(22) Anmeldetag: 27.06.2014
(51) Int. Cl.: A61N 1/05

(54) **Elektrodenleitung oder Elektrodenabschnitt einer Elektrodenleitung**
Electrode line or electrode portion of an electrode line
Ligne d'électrodes ou section d'électrodes d'une ligne d'électrodes

(30) Priorität: 26.08.2013 US 201361869767 P
(43) Veröffentlichungstag der Anmeldung: 04.03.2015
(73) Patentinhaber: BIOTRONIK SE & Co. KG, 12359 Berlin (DE)
(72) Erfinder: Rump, Jens, 12049 Berlin (DE); Büssing, Heinrich, 10317 Berlin (DE)
(74) Vertreter: Galander, Marcus

(56) Entgegenhaltungen:
- EP-A1- 1 574 181
- US-A1- 2005 084 672
- US-A1- 2008 177 353

## Beschreibung

Die Erfindung betrifft eine langgestreckte, implantierbare elektrische Leitung mit einer Endkomponente an einem Längsende der elektrischen Leitung, wobei die Endkomponente wenigstens eine elektrisch leitfähige, mit der elektrischen Leitung elektrisch verbundene Elektrodenfläche aufweist.

Solche langgestreckten, implantierbaren elektrischen Leitungen sind beispielsweise als Elektrodenleitungen, wie Stimulationselektrodenleitungen für implantierbare Herzschrittmacher oder Defibrillatoren, bekannt. Solche Elektrodenleitungen haben typischer Weise ein proximales Ende mit einem elektrischen Anschluss zum Anschließen der Elektrodenleitung an ein Stimulationsgerät, wie einen Herzschrittmacher oder Defibrillator, sowie ein distales Ende, an dem typischer Weise Stimulations- oder auch Abführelektroden angeordnet sind. Häufig ist am distalen Ende einer Elektrodenleitung ein Elektrodenkopf angeordnet, der eine sogenannte Tipp-Elektrode bildet. Mit geringem Abstand von der Tipp-Elektrode können auch eine oder mehrere Ringelektroden vorgesehen sein.

Aus der europäischen Patentanmeldung EP 0 787 507 ist eine Stimulationselektrodenleitung bekannt, bei der eine metallische Elektrode vollständig mit einer Isolierschicht überzogen ist. Als Metall der Elektrode ist Titan genannt und die Isolierschicht auf der Elektrode ist Titanoxid mit einer Schichtdicke in der Größenordnung von 1 µm. Alternativ ist als Metall der Elektrode Aluminium genannt und als Material der Isolierschicht Aluminiumoxid. Die europäische Patentanmeldung EP1 574 181 offenbart eine langgestreckte, implantierbare elektrische Leitung mit einer Endkomponente an einem Längsende der elektrischen Leitung, wobei die Endkomponente wenigstens eine elektrisch leitfähige, mit der elektrischen Leitung elektrisch verbundene Elektrodenfläche aufweist, wobei die Endkomponente eine Verbundkomponente ist, die wenigstens eine dünne Metallschicht mit einer Schichtdicke unter 1mikrometer aufweist, welche auf elektrisch isolierendes Material aufgebracht ist, mit der elektrischen Leitung leitend verbunden und auf ihrer Außenseite von wenigstens einer äußeren isolierenden Schicht nahezu vollständig oder vollständig bedeckt ist.

Der Erfindung liegt die Aufgabe zugrunde, eine alternative und möglichst verbesserte langgestreckte elektrische Leitung der eingangs genannten Art zu schaffen.

Erfindungsgemäß wird diese Aufgabe dadurch gelöst, dass die Endkomponente eine Verbundkomponente ist, die wenigstens eine dünne Metallschicht mit einer Schichtdicke unter 1 µm aufweist. Die dünne Metallschicht ist dabei auf elektrisch isolierendes Material der Verbundkomponente aufgebracht. Außerdem ist die dünne Metallschicht mit der elektrischen Leitung leitend verbunden und kann daher beispielsweise als Elektrodenfläche dienen. Auf ihrer Außenseite ist die dünne Metallschicht von wenigstens einer äußeren keramischen Schicht nahezu vollständig oder vollständig bedeckt. Im Idealfall ist die dünne Metallschicht dabei vollständig von der äußeren keramischen Schicht bedeckt und durch diese gegenüber der Umgebung elektrisch isoliert. Jedoch haben keramische Schichten neben vielen Vorteilen den Nachteil, dass sie kleine Löcher oder Fehlstellen, sogenannte Pinholes, aufweisen können, die dazu führen können, dass die dünne Metallschicht nicht mehr galvanisch von der Umgebung der Verbundkomponente getrennt ist.

Der Vorteil einer äußeren keramischen Schicht ist, dass diese auch bei geringer Dicke mechanisch und chemisch stabil ist. Ein Nachteil ist, dass keramische Schichten empfindlich gegenüber elektrischen Effekten, wie Funkenerosion, sind. Insbesondere führen kleine Fehlstellen in der Beschichtung (die Pinholes) bei elektrischer Belastung zur Ausbildung von Plasmakanälen. Diese Plasmakanäle erzeugen bei Entladung Temperaturspitzen, die Beschädigungen sowohl der isolierenden Schicht als auch der Trägerschicht zur Folge haben. Jede Beschädigung dieser Art kann zur Fehlfunktion der Elektrode beim Abfühlen (Sensing) oder Stimulieren (Pacing) führen. Eine bloße Vergrößerung der Schichtdicke der keramischen Schicht erhöht deren Langzeitstabilität nicht, da sich durch Vergrößerung der Schichtdicke Pinholes nicht schließen oder vermeiden lassen.

Die Erfinder haben diese den grundsätzlichen Vorteilen gegenüberstehenden Nachteile keramischer Isolierschichten erkannt und begegnen diesen dadurch, dass die Metallschicht dünn ausgebildet ist, so dass sie im Falle eines Pinhole infolge der ursprünglich als problematisch erkannten elektrischen Entladung verdampft, also durch Funkenerosion abgetragen wird, so dass die sich nicht im Bereich des Pinhole befindende übrige dünne Metallschicht schließlich wieder vollständig von der Umgebung der Verbundkomponente elektrisch isoliert ist.

Geeignete Schichtdicken für die äußere keramische Schicht sind vorzugsweise kleiner als 10 µm und vorzugsweise größer als 1 µm. Die Metallschicht hat vorzugsweise eine Schichtdicke kleiner als 100 nm, beispielsweise in der Größenordnung von 10 nm.

Das elektrisch isolierende Material, auf das die dünne Metallschicht aufgebracht ist, ist vorzugsweise ebenfalls ein keramisches Material. Insbesondere kann das elektrisch isolierende Material eine Isolierschicht sein, die auf einen metallischen Grundkörper aufgetragen ist. Besteht diese Isolierschicht ebenfalls aus keramischem Material - ist sie also ebenfalls eine keramische Schicht -, kann sie zwar ebenso wie die äußere keramische Schicht Pinholes aufweisen, die zur Folge haben können, dass die dünne Metallschicht den metallischen Grundkörper elektrisch kontaktiert. Jedoch ist es äußerst unwahrscheinlich, dass ein Pinhole in der Isolierschicht unter der dünnen Metallschicht sich an gleicher Stelle befindet wie ein Pinhole in der äußeren keramischen Schicht. Damit wäre ein Pinhole in der das elektrisch isolierende Material bildenden Schicht, auf das die Metallschicht aufgebracht ist, unkritisch. Um die Sicherheit noch weiter zu vergrößern, können mehrere Isolierschichten und dünne Metallschichten im Wechsel aufeinander folgen, so dass die Verbundkomponente mehrere dünne Metallschichten und dazwischenliegende Isolierschichten aufweist, wobei diese Isolierschichten vorzugsweise keramische Schichten sind. Die Metallschicht oder die Metallschichten sind dabei immer dünner als die entsprechenden Keramikschichten.

Ein geeignetes Material für die keramische Schicht oder die keramischen Schichten sind beispielsweise Siliziumoxid oder Siliziumcarbid, oder auch ein Metalloxid, wie Aluminiumoxid oder Tantaloxid. Die dünne Metallschicht kann beispielsweise eine Titanschicht sein. Ebenso kann das Material eines gegebenenfalls vorhandenen metallischen Grundkörpers Titan sein.

Die dünne Metallschicht, und im Falle mehrerer dünner Metallschichten insbesondere die äußerste dünne Metallschicht, wirkt dabei als Elektrodenfläche, und die langgestreckte elektrische Leitung ist vorzugsweise eine Stimulationselektrodenleitung, insbesondere für ein implantierbares Stimulationsgerät, wie beispielsweise einen Herzschrittmacher oder Defibrillator.

Die Verbundkomponente der langgestreckten elektrischen Leitung stellt einen grundsätzlich auch unabhängig von der übrigen langgestreckten elektrischen Leitung herstellbaren Gegenstand dar, der auch als selbständig schutzfähig betrachtet wird.

Die erfindungsgemäße elektrische Leitung und insbesondere ihre Verbundkomponente wird vorzugsweise mit einem Herstellverfahren erzeugt, bei dem eine oder mehrere keramische Schichten mittels physikalischer Gasabscheidung (PVD, Physical Vapor Deposition) aufgebracht werden. Zusätzlich oder alternativ kann das Herstellverfahren auch den Verfahrensschritt enthalten, dass eine oder mehrere Metallschichten mittels chemischer Gasabscheidung (CVD, Chemical Vapor Deposition) aufgebracht werden.

Der Schritt der Funkenerosion zum Verdampfen der dünnen Metallschicht im Bereich von Pinholes der äußeren keramischen Schicht kann ebenfalls bereits bei der Herstellung der erfindungsgemäßen elektrischen Leitung bzw. der erfindungsgemäßen Verbundkomponente durchgeführt werden.

Die erfindungsgemäße Verbundkomponente stellt vorzugsweise einen Elektrodenkopf für eine Stimulationselektrodenleitung dar.

Die Erfindung soll nun anhand eines Ausführungsbeispiels mit Bezug auf die Figuren näher erläutert werden. Von den Figuren zeigt:
- Fig. 1:: Eine schematische Ansicht einer Stimulationselektrodenleitung;
- Fig. 2:: eine schematische Darstellung einer Verbundkomponente mit einem Grundkörper aus elektrisch nicht leitendem Material und einer dünnen Metallschicht sowie einer äußeren keramischen Schicht;
- Fig. 3:: eine Variante einer Verbundkomponente mit mehreren Metallschichten und mehreren keramischen Schichten.

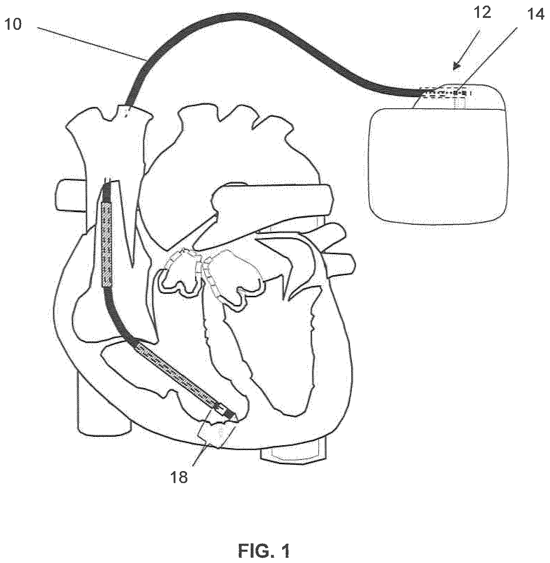

Fig. 1 zeigt eine Elektrodenleitung 10 mit einem proximalen Ende 12, an dem eine elektrische Steckverbindung 14 vorgesehen ist. Die Elektrodenleitung 10 besitzt ein distales Ende 16 mit einem Elektrodenkopf 18, der als Verbundkomponente ausgebildet ist. Die Elektrodenleitung 10 ist eine langgestreckte elektrische Leitung und besitzt wenigstens einen elektrischen Leiter, der sich vom proximalen zum distalen Ende erstreckt und somit eine elektrische Verbindung zwischen dem Elektrodenkopf 18 und dem elektrischen Anschluss 14 herstellt. Der elektrische Leiter besteht vorzugsweise aus Titan. Genauso besitzt der Elektrodenkopf 18 vorzugsweise einen Grundkörper aus Titan. Die Elektrodenleitung ist damit Röntgen-transparent.

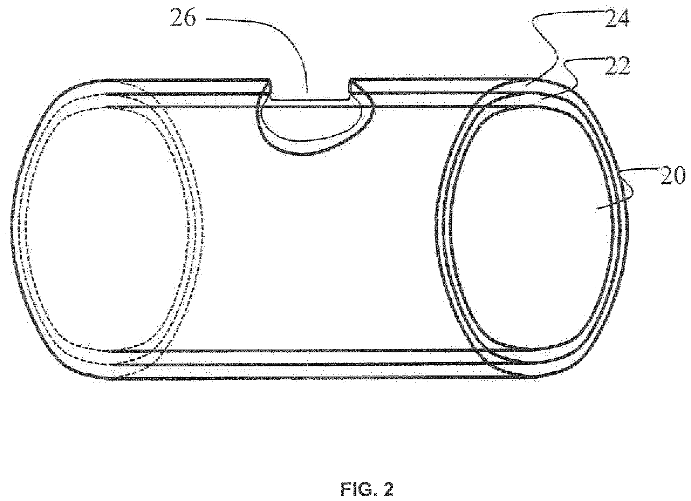

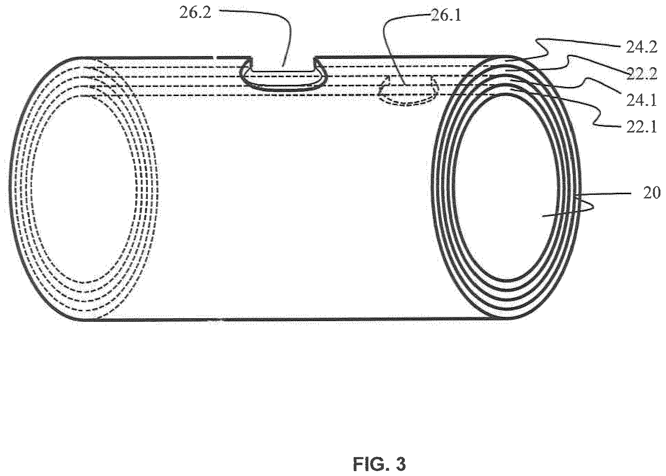

Figuren 2 und 3 zeigen schematisch den Aufbau des Elektrodenkopfes als Verbundkomponente. Auf einen isolierenden Grundkörper 20 ist wenigstens eine Metallschicht 22 aufgebracht. Diese ist auf der Außenseite im Wesentlichen vollständig von einer keramischen Schicht 24 bedeckt, die als Isolierschicht dient.

Nicht dargestellt ist, dass der elektrisch isolierende Grundkörper 20 selbst aus mehreren Komponenten bestehen kann und beispielsweise vorzugsweise aus einem metallischen Grundkörper besteht, auf den eine innere Isolierschicht, vorzugsweise ebenfalls eine keramische Schicht, aufgebracht ist. Zwischen dem in Fig. 2 dargestellten Grundkörper 20 und der Metallschicht 22 kann sich also noch eine Isolierschicht befinden.

Wie in Fig. 2 beispielshaft dargestellt ist, besitzt die äußere keramische Schicht 24 eine Fehlstelle 26, so dass die Metallschicht 22 im Bereich der Fehlstelle 26 nicht mehr durch die äußere keramische Schicht 24 galvanisch von der Umgebung der Verbundkomponente 18 getrennt ist. Im Bereich der Fehlstelle 26 kann es daher zu einer Funkenerosion kommen, die zum Verdampfen der Metallschicht 22 im Bereich der Fehlstelle 26 führt, so dass im Ergebnis die verbleibende Metallschicht 22 wieder galvanisch von der Umgebung der Verbundkomponente 18 getrennt ist.

Fig. 3 zeigt eine Verbundkomponente ähnlich der Verbundkomponente aus Fig. 2 mit dem Unterschied, dass bei der Verbundkomponente aus Fig. 3 mehrere Metallschichten und mehrere keramische Schichten aufgebracht sind. Eine Fehlstelle 26.1 in einer zwischen zwei Metallschichten 22.1 und 22.2 liegenden Isolierschicht 24.1 ist im Ergebnis bedeutungslos, weil diese Fehlstelle von einer zweiten Metallschicht 22.2 und der äußeren keramischen Schicht 24.2 bedeckt ist. Mit der ebenfalls beispielhaft dargestellten Fehlstelle 26.2 verhält es sich hingegen genauso wie mit der Fehlstelle 26 aus dem Ausführungsbeispiel in Fig. 2. Im Bereich der Fehlstelle 26.2 kommt es zur Funkenerosion und infolge dessen zum Verdampfen der Metallschicht 22.2 im Bereich der Fehlstelle 26.2.

Die Isolierung an Kopfelektroden oder -hülsen an implantierbaren Elektroden kann durch chemische, elektrische und/oder mechanische äußere Einwirkungen erodieren. Bei einer Sandwichbauweise von abwechselnd metallischen und keramischen Schichten ist die mechanische Festigkeit der Isolierung durch die Keramik gewährleistet. Auftretende Fehlstellen der keramischen Schicht können durch die elektrisch wirksamen Metallschichten mittels Funkenerosion behoben werden. Die Langzeitstabilität wird dadurch erhöht. Es ergibt sich eine isolierende Dünnschicht (<10 µm), die weitestgehend resistent gegen mechanische, chemische und elektrische Belastung und insbesondere chemisch inert und stabil gegenüber mechanischen und elektrischen Einflüssen ist. Dies sichert eine langandauernde Stabilität der elektrischen Eigenschaften (Gleichstromwiderstand) der Beschichtung und verhindert ungewollte Erosion.

Zur Herstellung einer röntgendurchsichtigen Titan-Kopfelektrode oder -hülse für eine implantierbare Elektrodenleitung wird auf einen Grundkörper aus Titan durch PVD-Verfahren eine 1-2µm dicke Silizium-Carbid (SiC) Schicht aufgebracht. Durch anschließendes Bedampfen mit Titan wird die SiC-Schicht metallisiert bis eine wenige Nanometer dicke Titanschicht < 500nm entsteht. Pinholes werden dabei durch das Metall aufgefüllt und die metallene Dünnschicht (Metallschicht) ist elektrisch mit dem Grundkörper aus Titan verbunden. Über die Metallschicht wird durch Sputtern (mindestens) eine weitere SiC-Schicht aufgetragen. Dabei entstehende Pinholes befinden sich mit nur sehr geringer Wahrscheinlichkeit über den Pinholes der unteren SiC-Schicht. Die Wahrscheinlichkeit kann weiter verringert werden indem die Anzahl der Lagen auf 3 oder mehr erhöht wird.

Mit Anlegen einer elektrischen Spannung an den Schichtverbund wird an Pinholes der äußeren SiC-Schicht Funkenerosion provoziert und die dort befindliche dünne Metallisierung verdampft.

Äußeres Pinhole und innere Kopfhülse sind nach diesem Verfahren elektrisch voneinander getrennt, wodurch eine Art von Selbstheilung erreicht wird.

Die Vorteile einer mit mehreren Metall- und Isolierschichten versehenen Verbundkomponente ergeben sich aus dem Vergleich der Figuren 2 und 3. Wird beispielsweise im Lauf der Verwendung der Elektrodenleitung die äußere keramische Schicht 24 durch mechanische, chemische oder elektrische Beanspruchung verletzt (Fehlstelle 26) ist die darunterliegende äußere Metallschicht 22 galvanisch der Umgebung der Verbundkomponente (z.B. ein Elektrolyt wie Blut) verbunden und die Funktion der Elektrode wird beeinträchtigt.

Besitzt die Verbundkomponente mehrere Metallschichten 22.1 und 22.2 und keramische Schichten 24.1 und 24.2 hat eine Fehlstelle 26.2 in der äußern keramischen Schicht 26.2 lediglich zur Folge dass die innere Metallschicht 22.1 durch die mittlere keramische Schicht 24.1 und die äußere Metallschicht 22.2 vor dem Eindringen von Elektrolyt und einem weiteren Aufbruch geschützt ist.

Vorteil der vorgestellten erfindungsmäßigen Lösung ist eine hohe Festigkeit einer dünnen Isolierung von Elektroden von unter 10 µm verbunden mit einer hohen Fertigungstoleranz. Fehlstellen der Keramikschicht, die die Funktionalität der Elektrode beeinträchtigen, können nachträglich elektrisch behoben werden.

## Patentansprüche

1. Langgestreckte, implantierbare elektrische Leitung (10) mit einer Endkomponente (18) an einem Längsende der elektrischen Leitung, wobei die Endkomponente (18) wenigstens eine elektrisch leitfähige, mit der elektrischen Leitung elektrisch verbundene Elektrodenfläche (22) aufweist und weiterhin die Endkomponente (18) eine Verbundkomponente ist, die wenigstens eine dünne Metallschicht (22) mit einer Schichtdicke unter 1µm aufweist, welche auf elektrisch isolierendes Material aufgebracht ist, mit der elektrischen Leitung leitend verbunden und auf ihrer Außenseite von wenigstens einer äußeren isolierenden, keramischen Schicht (24) nahezu vollständig oder vollständig bedeckt ist,
wobei die Metallschicht (22) oder -schichten (22.1, 22.2) dünner sind als die keramische Schicht (24) oder Schichten (24.1, 24.2).

2. Elektrische Leitung (10) gemäß Anspruch 1, **dadurch gekennzeichnet, dass** die isolierende Schicht (24) eine Schichtdicke kleiner 10µm hat.

3. Elektrische Leitung (10) gemäß Anspruch 1 oder 2, **dadurch gekennzeichnet, dass** die isolierende Schicht (24) eine Schichtdicke größer 1µm hat.

4. Elektrische Leitung gemäß einem der Ansprüche 1 bis 3, **dadurch gekennzeichnet, dass** die Metallschicht (22) eine Schichtdicke kleiner 100 nm hat

5. Elektrische Leitung (10) gemäß einem der Ansprüche 1 bis 4, **dadurch gekennzeichnet, dass** die Verbundkomponente (18) einen Grundkörper (20) aus Metall besitzt, auf den eine innere Isolierschicht aufgetragen ist, auf welche die dünne Metallschicht (22) aufgetragen ist.

6. Elektrische Leitung (10) gemäß einem der Ansprüche 1 bis 5, **dadurch gekennzeichnet, dass** die dünne Metallschicht die Elektrodenfläche bildet und durch die keramische Schicht von der Umgebung der Verbundkomponente elektrisch isoliert ist.

7. Elektrische Leitung (10) gemäß einem der Ansprüche 1 bis 6, **dadurch gekennzeichnet, dass** die Verbundkomponente (18) mehrere dünne Metallschichten (22.1, 22.2) und dazwischenliegende Isolierschichten (24.1) aufweist.

8. Elektrische Leitung (10) gemäß Anspruch 7, **dadurch gekennzeichnet, dass** die Isolierschichten (24.1, 24.2) keramische Schichten sind.

9. Elektrische Leitung gemäß einem der Ansprüche 1 bis 8, **dadurch gekennzeichnet, dass** die keramische Schicht (24) oder die keramischen Schichten (24.1, 24.2) Siliziumoxid, Siliziumcarbid oder ein Metalloxid wie Alumiumoxid oder Tantaloxid enthalten oder aus solchem keramischen Material bestehen.

10. Elektrische Leitung (10) gemäß einem der Ansprüche 1 bis 9, **dadurch gekennzeichnet, dass** die Metallschichten (22) sowie die Isolierschichten (24) und die äußere keramische Schicht (24) zusammen eine Dicke von weniger als 10 µm haben.

11. Elektrische Leitung (10) gemäß einem der Ansprüche 1 bis 10, **dadurch gekennzeichnet, dass** die elektrische Leitung (10) eine Stimulationselektrodenleitung ist.

12. Verfahren zum Herstellen einer elektrischen Leitung gemäß einem der Ansprüche 1 bis 11, **dadurch gekennzeichnet, dass** eine oder mehrere keramische Schichten (24) mittels physikalischer Gasabscheidung (PVD) aufgebracht werden.

13. Verfahren zum Herstellen einer elektrischen Leitung (10) gemäß einem der Ansprüche 1 bis 11, **dadurch gekennzeichnet, dass** eine oder mehrere Metallschichten (22) mittels chemischer Gasabscheidung (CVD) aufgebracht werden.

14. Verfahren zum Herstellen einer elektrischen Leitung (10) gemäß einem der Ansprüche 1 bis 11, **dadurch gekennzeichnet, dass** durch Anlegen elektrischer Spannung eine Funkenerosion der im Bereich von Fehlstellen der äußeren keramischen Schicht (24) ggf. freiliegenden Metallschicht (22) durchgeführt wird.

## Claims

1. An elongate, implantable electrical line (10) having an end component (18) at one longitudinal end of the electrical line, wherein the end component (18) has at least one electrically conductive electrode surface (22) electrically connected to the electrical line and in addition the end component (18) is a composite component that has at least one thin metal layer (22) with a layer thickness less than 1 µm, which is applied to electrically insulating material, is conductively connected to the electrical line, and is covered completely or practically completely on its outer face by at least one outer insulating, ceramic layer (24), wherein the metal layer (22) or layers (22.1, 22.2) are thinner than the ceramic layer (24) or layers (24.1, 24.2).

2. The electrical line (10) according to claim 1, **characterised in that** the insulating layer (24) has a layer thickness less than 10 µm.

3. The electrical line (10) according to claim 1 or 2, **characterised in that** the insulating layer (24) has a layer thickness greater than 1 µm.

4. The electrical line (10) according to any one of claims 1 to 3, **characterised in that** the metal layer (22) has a layer thickness less than 100 nm.

5. The electrical line (10) according to any one of claims 1 to 4, **characterised in that** the composite component (18) has a main body (20) made of metal, to which an inner insulating layer is applied, to which the thin metal layer (22) is applied.

6. The electrical line (10) according to any one of claims 1 to 5, **characterised in that** the thin metal layer forms the electrode surface and is electrically insulated by the ceramic layer from the environment surrounding the composite component.

7. The electrical line (10) according to any one of claims 1 to 6, **characterised in that** the composite component (18) has a plurality of thin metal layers (22.1, 22.2) and insulating layers (24.1) arranged therebetween.

8. The electrical line (10) according to claim 7, **characterised in that** the insulating layers (24.1, 24.2) are ceramic layers.

9. The electrical line according to any one of claims 1 to 8, **characterised in that** the ceramic layer (24) or the ceramic layers (24.1, 24.2) contain silicon oxide, silicon carbide or a metal oxide, such as aluminium oxide or tantalum oxide, or consist of such a ceramic material.

10. The electrical line (10) according to any one of claims 1 to 9, **characterised in that** the metal layers (22) and also the insulating layers (24) and the outer ceramic layer (24) together have a thickness of less than 10 µm.

11. The electrical line (10) according to any one of claims 1 to 10, **characterised in that** the electrical line (10) is a stimulation electrode line.

12. A method for producing an electrical line according to any one of claims 1 to 11, **characterised in that** one or more ceramic layers (24) is/are applied by means of physical vapour deposition (PVD).

13. A method for producing an electrical line (10) according to any one of claims 1 to 11, **characterised in that** one or more metal layers (22) is/are applied by means of chemical vapour deposition (CVD).

14. A method for producing an electrical line (10) according to any one of claims 1 to 11, **characterised in that**, by applying electrical voltage, a spark erosion of the metal layer (22) potentially exposed in the region of imperfections in the outer ceramic layer (24) is carried out.

## Revendications

1. Ligne électrique implantable (10) allongée avec une composante terminale (18) à une extrémité longitudinale de la ligne électrique, où la composante terminale (18) présente au moins une surface d'électrode (22) conductrice électriquement, reliée électriquement avec la ligne électrique, et en outre la composante terminale (18) est une composante composite qui présente au moins une couche métallique (22) mince avec une épaisseur de couche inférieure à 1 µm, laquelle est rapportée sur un matériau isolant électriquement, est reliée de manière conductrice avec la ligne électrique et est recouverte presque totalement ou totalement sur sa face extérieure avec au moins une couche de céramique (24) isolante extérieure,
où la couche (22) ou les couches (22.1, 22.2) métalliques sont plus minces que la couche (24) ou les couches (24.1, 24.2) de céramique.

2. Ligne électrique (10) selon la revendication 1, **caractérisée en ce que** la couche isolante (24) a une épaisseur de couche inférieure à 10 µm.

3. Ligne électrique (10) selon la revendication 1 ou 2, **caractérisée en ce que** la couche isolante (24) a une épaisseur de couche supérieure à 1 µm.

4. Ligne électrique selon l'une des revendications 1 à 3, **caractérisée en ce que** la couche métallique (22) a une épaisseur de couche inférieure à 100 nm.

5. Ligne électrique (10) selon l'une des revendications 1 à 4, **caractérisée en ce que** la composante composite (18) possède un corps de base (20) en métal, sur lequel est rapportée une couche d'isolant interne, sur laquelle est rapportée la couche métallique (22) mince.

6. Ligne électrique (10) selon l'une des revendications 1 à 5, **caractérisée en ce que** la couche métallique mince forme la surface d'électrode et est isolée électriquement de l'environnement de la composante composite par la couche de céramique.

7. Ligne électrique (10) selon l'une des revendications 1 à 6, **caractérisée en ce que** la composante composite (18) présente plusieurs couches métalliques (22.1, 22.2) minces et des couches isolantes (24.1) se situant entre celles-ci.

8. Ligne électrique (10) selon la revendication 7, **caractérisée en ce que** les couches isolantes (24.1, 24.2) sont des couches de céramique.

9. Ligne électrique selon l'une des revendications 1 à 8, **caractérisée en ce que** la couche de céramique (24) ou les couches de céramiques (24.1, 24.2) contiennent de l'oxyde de silicium, du carbure de silicium ou un oxyde métallique comme l'oxyde d'aluminium ou l'oxyde de tantale ou sont constituées d'un tel matériau céramique.

10. Ligne électrique (10) selon l'une des revendications 1 à 9, **caractérisée en ce que** les couches métalliques (22) ainsi que les couches isolantes (24) et la couche de céramique (24) extérieure ont ensemble une épaisseur de moins de 10 µm.

11. Ligne électrique (10) selon l'une des revendications 1 à 10, **caractérisée en ce que** la ligne électrique (10) est une ligne d'électrode de stimulation.

12. Procédé de fabrication d'une ligne électrique selon l'une des revendications 1 à 11, **caractérisé en ce qu'**une ou plusieurs couches de céramique (24) sont rapportées au moyen d'un dépôt physique en phase vapeur (PVD).

13. Procédé de fabrication d'une ligne électrique (10) selon l'une des revendications 1 à 11, **caractérisé en ce qu'**une ou plusieurs couches métalliques (22) sont rapportées au moyen d'un dépôt chimique en phase vapeur (CVD).

14. Procédé de fabrication d'une ligne électrique (10) selon l'une des revendications 1 à 11, **caractérisé en ce que**, par l'application d'une tension électrique, une électroérosion de la couche métallique (22) éventuellement libre dans les zones de défauts de la couche de céramique (24) externe est réalisée.
